# EUROPÄISCHE PATENTSCHRIFT

(11) **EP 0 370 454 B1**
(45) Veröffentlichungstag und Bekanntmachung des Hinweises auf die Patenterteilung: **20.04.1994**
(21) Anmeldenummer: 89121499.1
(22) Anmeldetag: 21.11.1989
(51) Int. Cl.: C07K 5/06, C07D 233/64, A61K 37/64, A61K 31/415

(54) **Renin-hemmende Aminodiol-Derivate**
Renin-inhibiting amino diol derivatives
Dérivés de diols aminés inhibant la rénine

(30) Priorität: 24.11.1988 DE 3839559
(43) Veröffentlichungstag der Anmeldung: 30.05.1990
(73) Patentinhaber: HOECHST AKTIENGESELLSCHAFT, 65926 Frankfurt am Main (DE)
(72) Erfinder: Henning, Rainer, Dr., D-6234 Hattersheim am Main (DE); Urbach, Hansjörg, Dr., D-6242 Kronberg/Taunus (DE); Ruppert, Dieter, Dr., D-6242 Kronberg/Taunus (DE); Schölkens, Bernward, Dr., D-6233 Kelkheim (Taunus) (DE)

(56) Entgegenhaltungen:
- EP-A- 0 255 082
- EP-A- 0 309 766
- WO-A-88/05050
- WO-A-89/10751
- PEPTIDES, Structure an Function, Proceedings of the Ninth American Peptide Symposium, pages 751-754, Pierce Chemical Company, Rockford, Illinois, US; M.G. BOCK et al.: "Renin inhibitors. Synthesis and biological activity of statine- and achpa-containing peptides having polar end groups"

## Beschreibung

Aus den europäischen Patentanmeldungen EP-A 184 855, 189 203, 202 571, 229 667, 230 266 sowie 237 202 und der International Patent Application WO 87/05302 sind Aminodiol-Derivate mit reninhemmender Wirkung bekannt.

Weiterhin sind reninhemmende Aminodiol-Derivate in Biochem. Biophys. Res. Commun. 132, 155 - 161 (1985), in Biochem. Biophys. Res. Commun. 146, 959 - 963 (1987), in FEBS Lett. 230, 38 - 42 (1988) und in J. Med. Chem. 30, 976 - 982 (1987) beschrieben.

Überraschenderweise wurde nun gefunden, daß solche Verbindungen, die sich von den in den genannten Dokumenten beschriebenen dadurch unterscheiden, daß sie am C-Terminus einen Heterocyclus tragen, hochwirksame Reninhemmer in vitro und in vivo sind und gegenüber den bekannten Verbindungen vorteilhafte Eigenschaften aufweisen.

Die Erfindung betrifft daher Verbindungen der Formel I 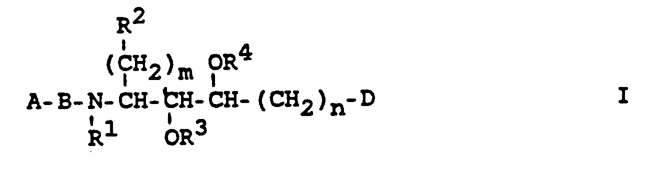
in welcher A einen Rest der Formel II 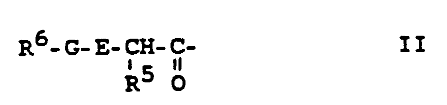
bedeutet, worin
- R⁵: Phenyl, 2-Thienyl, 2-Pyridyl, 1-Naphthyl oder Benzyl, 2-Thienylmethyl, 2-Pyridylmethyl, 1-Naphthylmethyl, die jeweils gegebenenfalls durch Hydroxy, Dihydroxy, Methoxy, Dimethoxy, F oder Cl substituiert sind, bedeutet;
- E: eine CH₂-Gruppe, eine -NH-Gruppe oder eine N-(CH₃)-Gruppe bedeutet;
- G: einen Rest der Gruppe S, SO, SO₂, O, CO oder CS bedeutet;
- R⁶: Methyl, Ethyl, Isopropyl, tert.-Butyl, Isobutyl, 2-Hydroxyethyl, 2-Methoxyethyl, Carboxymethyl, 2-Carboxyethyl, Methoxycarbonylmethyl, 2-Methoxycarbonylethyl, Ethoxycarbonylmethyl, 2-Ethoxycarbonylethyl, Carbamoylmethyl, 2-Carbamoylethyl, 2-Aminoethyl, 2-Dimethylaminoethyl, 2-Morpholinoethyl, Aminopropyl, Aminoisobutyl, Methylaminosobutyl, Dimethylaminoisobutyl, 2-Piperidinoethyl, Cyclopropyl, Cyclobutyl, Cyclopentyl oder Cyclohexyl, Phenyl, 1- oder 2-Naphthyl, o-, m- oder p-Methylphenyl, o-, m- oder p-Hydroxyphenyl oder o-, m- oder p-Aminophenyl, Benzyl, 2-Phenylethyl oder α- oder β-Naphthylmethyl, unsubstituiertes oder substituiertes Heteroaryl, z.B. 2- oder 3-Pyrrolyl, 2-Furyl, 2-Thienyl, 2- oder 4-Imidazolyl, 1-Methyl-2-, -4- oder -5-Imidazolyl, 1,3-Thiazol-2-yl, 2-, 3- oder 4-Pyridyl, 1-Oxido-2-, 3- oder 4-pyridinio, 2-Pyrazinyl, 2-, 4- oder 5-Pyrimidinyl, 2-, 3- oder 4-Chinolyl, 1-, 3- oder 4-Isochinolyl oder 2-Benzoxazolyl, Methoxy, Ethoxy oder n-Butoxy oder Amino als Teil eines fünf- oder sechsgliedrigen Ringes enthaltend ein Stickstoffatom und gewünschtenfalls ein Sauerstoffatom, z.B. 1-Pyrrolidinyl, 1-Piperidinyl oder 4-Morpholinyl;
wobei der heterocyclische Ring auch
durch ein oder zwei Reste der Gruppe (C₁-C₄)-Alkyl, (C₁-C₄)-Alkoxy, (C₁-C₄)-Alkoxycarbonyl, Hydroxy, Halogen, Amino, Mono- oder Di-(C₁-C₄)-alkylamino substituiert sein kann
- B: für einen bivalenten Rest aus der Gruppe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, 2,4-Diaminobuttersäure, Arginin, Norvalin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norleucin, Valin, Alanin, Cystein, S-Methylcystein, N-Methyl-Histidin, Benzodioxol-5-yl-alanin, 1,2,3,4-Tetrahydroisochinolin-3-carbonsäure, Homophenylalanin, 2-Amino-4-(2-thienyl)-buttersäure, (Z)-Dehydrophenylalanin, (E)-Dehydrophenylalanin, Dioxolan-1,3-yl-3-alanin steht;
- R¹: Wasserstoff bedeutet;
- R²: Isopropyl oder Cyclohexyl bedeutet;
- R³ und R⁴: gleich oder verschieden und voneinander unabhängig Wasserstoff, Acetoxymethyl, Acetoxyethyl, Pivaloyloxymethyl, Pivaloyloxyethyl, 2,2-Dimethylbutyryloxymethyl, Ethoxycarbonyloxymethyl, Ethoxycarbonyloxyethyl, tert.-Butoxycarbonyloxymethyl oder tert.-Butoxycarbonyloxyethyl bedeutet oder worin
- R³ und R⁴: zusammen mit den sie tragenden Sauerstoffatomen einen Dioxolanon-, Dioxandion-, Dimethyldioxolan-, Phenyldioxolan- oder Cyclohexylidendioxolanring bilden;
- D: einen 2-, 3- oder 4-Pyridinrest,
- m: 1 bedeutet und
- n: 0, 1, 2 oder 3 bedeutet,
sowie deren physiologisch verträgliche Salze.

Die Chiralitätszentren in den Verbindungen der Formel I können die R-, S- oder R,S-Konfiguration aufweisen.

Alkyl kann geradkettig oder verzweigt sein. Entsprechendes gilt für davon abgeleitete Reste, wie z.B. Alkoxy, Alkylthio, Alkylamino, Dialkylamino und Alkanoyl.

Unter Cycloalkyl werden auch alkylsubstituierte Reste, wie z.B. 4-Methylcyclohexyl oder 2,3-Dimethylcyclopentyl verstanden.

(C₆-C₁₄)-Aryl ist beispielsweise Phenyl, Naphthyl, Biphenylyl oder Fluorenyl; bevorzugt ist Phenyl. Entsprechendes gilt für (C₆-C₁₄)-Aryl-(C₁-C₄)-alkyl. Bevorzugte Reste dieser Art sind z.B. Benzyl, α- und β-Naphthylmethyl, Halobenzyl und Alkoxybenzyl.

Ein Rest Het im Sinne vorstehender Definition ist beispielsweise Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder ein benzannelliertes, cyclopenta-, cyclohexa- oder cyclohepta-annelliertes Derivat dieser Reste.

Bevorzugte Reste Het sind 2- oder 3-Pyrrolyl, Phenyl-pyrrolyl, z.B. 4- oder 5-Phenyl-2-pyrrolyl, 2-Furyl, 2-Thienyl, 4-Imidazolyl, Methyl-imidazolyl, z.B. 1-Methyl-2-, 4- oder 5-imidazolyl, 1,3-Thiazol-2-yl, 2-, 3- oder 4-Pyridyl, 1-Oxido-2-, 3- oder 4-pyridinio, 1- oder 2-Pyrazinyl, 2-, 4- oder 5-Pyrimidinyl, 4-Morpholinyl, 2-, 3- oder 5-Indolyl, substituiertes 2-Indolyl, z.B. 1-Methyl-, 5-Methyl-, 5-Methoxy, 5-Benzyloxy-, 5-Chlor- oder 4,5-Dimethyl-2-indolyl, 1-Benzyl-2- oder 3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, Benz[e]indol-2-yl, β-Carbolin-3-yl, 2-Oxazolinyl, 4-Alkyl-2-oxazolinyl oder 4,5-Dialkyloxazolinyl.

Unter Salzen von Verbindungen der Formel I sind insbesondere pharmazeutisch verwendbare oder nicht-toxische Salze zu verstehen.

Solche Salze werden beispielsweise von Verbindungen der Formel I, welche saure Gruppen, z.B. Carboxy, enthalten, mit Alkali- oder Erdalkalimetallen gebildet, wie Na, K, Mg und Ca, sowie mit physiologisch verträglichen organischen Aminen, wie z.B. Triethylamin und Tri-(2-hydroxy-ethyl)-amin.

Verbindungen der Formel I, welche basische Gruppen, z.B. eine Aminogruppe enthalten, bilden Salze mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure.

Die Erfindung betrifft weiterhin ein Verfahren zur Herstellung von Verbindungen der Formel I, das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Fragmente einer Verbindung der Formel I mit einer endständigen Carboxylgruppe besitzen die nachstehenden Formeln IIIa und IIIb:

A-OH (IIIa) A-B-OH (IIIb)

Fragmente einer Verbindung der Formel I mit einer endständigen Aminogruppe besitzen die nachstehenden Formeln IVa und IVb: 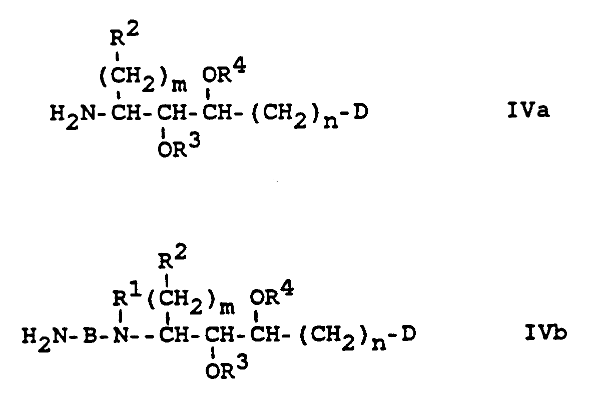
Methoden, die zur Herstellung einer Amidbindung geeignet sind, werden z.B. in Houben-Weyl, Methoden der organischen Chemie, Band 15/2; Bodanszky et al., Peptide synthesis, 2nd ed. (Wiley & Sons, New York 1976) oder Gross, Meienhofer, The Peptides. Analysis, synthesis, biology (Academic Press, New York 1979) beschrieben. Vorzugsweise werden die folgenden Methoden herangezogen: Aktivestermethode mit N-Hydroxy-succinimid als Esterkomponente, Kupplung mit einem Carbodiimid wie Dicyclohexylcarbodiimid oder mit Propanphosphonsäureanhydrid und die Gemischt-Anhydrid-Methode mit Pivaloylchlorid.

Die Herstellung der als Ausgangsverbindung verwendeten optisch aktiven Aminodiole der Formel V 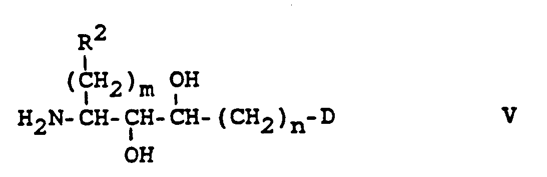
kann beispielsweise für den Fall, daß n 2 bedeutet, durch Umsetzung einer Verbindung der Formel VI mit einer Verbindung der Formel VII nach deren Deprotonierung erfolgen. 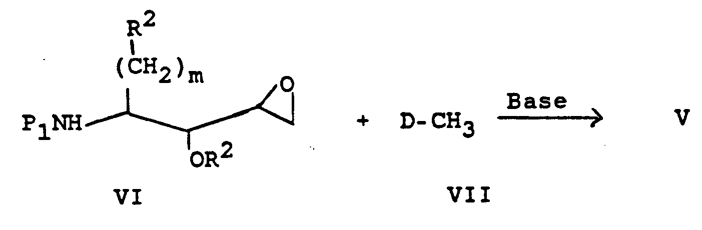
In der Formel VI bedeutet P₁ eine Urethanschutzgruppe, bevorzugt N-tert.-Butoxycarbonyl- und Benzyloxycarbonyl.

Als Basen zur Deprotonierung der Heteroarylalkyl-Komponente können Alkalimetallalkoholnte, wie Kalium-O-tert.-butylat, Natriummethylat, Alkalimetallhydride, wie Natrium- oder Kaliumhydrid, metallorganische Basen, wie n-Butyllithium, s-Butyllithium, Methyllithium oder Phenyllithium, Natriumamid sowie Alkalimetallsalze von organischen Stickstoffbasen, wie Lithiumdiisopropylamid verwendet werden.

Die Addition der Verbindungen der Formel VII an die genannten N-geschützten Epoxide erfolgt in einem gegenüber Basen inerten Lösungsmittel wie Ether, THF, Toluol, DMF, DMSO oder Dimethoxyethan.

Verbindungen der Formel VI sind aus EP-A 189 203 bekannt.

Weiterhin können die verschiedenen Isomeren von Verbindungen der Formel V nach dem nachfolgenden Schema 1 hergestellt werden:

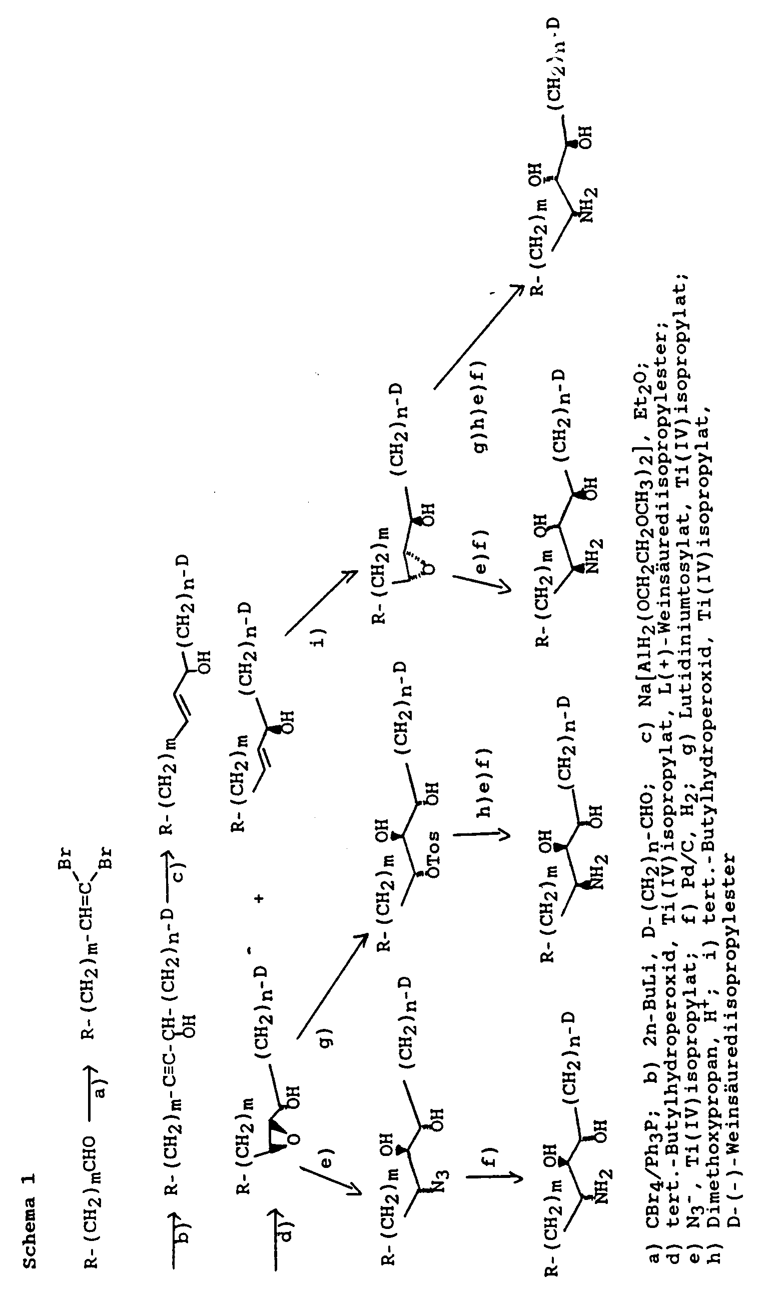

Falls der gewählte Syntheseweg zu Diastereomeren bezüglich der OR³ und OR⁴ tragenden Zentren führt, können diese in an sich bekannter Weise, beispielsweise durch fraktionierte Kristallisation oder durch Chromatographie getrennt werden. Die Überprüfung der Diastereomerenreinheit erfolgt mittels HPLC, die Enantiomerenreinheit kann in bekannter Weise durch Überführung in Mosher-Derivate überprüft werden (H. S. Mosher et al., J. Org. Chem. 34, 2543 (1969)).

In einer erhältlichen Verbindung der Formel I kann man eine Thiogruppe zu einer Sulfinyl- oder Sulfonylgruppe oder eine Sulfinylgruppe zu einer Sulfonylgruppe oxidieren.

Die Oxidation zur Sulfonylgruppe kann mit den meisten der üblichen Oxidationsmittel durchgeführt werden. Bevorzugt verwendet man solche Oxidationsmittel, die die Thiogruppe oder Sulfinylgruppe selektiv in Gegenwart anderer funktioneller Gruppen der Verbindung der Formel I, z.B. der Amidfunktion und der Hydroxygruppe, oxidieren, beispielsweise aromatische oder aliphatische Peroxycarbonsäuren, z.B. Perbenzoesäure, Monoperphthalsäure, m-Chlorperbenzoesäure, Peressigsäure, Perameisensäure oder Trifluorperessigsäure.

Die zur Herstellung von Verbindungen der Formel I erforderlichen Vor- und Nachoperationen wie Einführung und Abspaltung von Schutzgruppen sind literaturbekannt und sind z.B. in T.W. Greene, "Protective Groups in Organic Synthesis" beschrieben. Salze von Verbindungen der Formel I mit salzbildenden Gruppen werden in an sich bekannter Weise hergestellt, indem man z.B. eine Verbindung der Formel I mit einer basischen Gruppe mit einer stöchiometrischen Menge einer geeigneten Säure umsetzt. Stereoisomerengemische, insbesondere Diastereomerengemische, die bei Verwendung racemischer Säuren A oder B anfallen, können in an sich bekannter Weise durch fraktionierte Kristallisation oder durch Chromatographie getrennt werden.

Die erfindungsgemäßen Verbindungen der Formel I weisen enzymhemmende Eigenschaften auf; insbesondere hemmen sie die Wirkung des natürlichen Enzyms Renin. Renin ist ein proteolytisches Enzym aus der Klasse der Aspartyl-Proteasen welches als Folge verschiedener Stimuli (Volumendepletion, Natriummangel, β-Rezeptorenstimulation) von den juxtaglomerulären Zellen der Niere in den Blutkreislauf sezerniert wird. Dort spaltet es von dem aus der Leber ausgeschiedenen Angiotensinogen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin I, was eine verminderte Bildung von Angiotensin II zur Folge hat. Die Erniedrigung der Konzentration dieses aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin I in verschiedenen Systemen (Humanplasma, Schweine-Renin) gemessen. Hierzu wird z.B. Humanplasma, welches sowohl Renin als auch Angiotensinogen enthält, bei 37°C mit der zu testenden Verbindung inkubiert. Anschließend wird die Konzentration des während der Inkubation gebildeten Angiotensin I mit einem Radioimmunoassay gemessen. Die in der vorliegenden Erfindung beschriebenen Verbindungen der allgemeinen Formel I zeigen in verwendeten in-vitro-Tests Hemmwirkungen bei Konzentrationen von etwa 10⁻⁵ bis 10⁻¹⁰ Mol/l.

Renin-Hemmer bewirken an salzverarmten Tieren eine Blutdrucksenkung. Da sich menschliches Renin von dem Renin anderer Spezies unterscheidet, werden zum in-vivo-Test von Renin-Hemmern Primaten (Marmosets, Rhesus-Affen) herangezogen. Primaten-Renin und Human-Renin sind in ihrer Sequenz weitgehend homolog. Durch i.v. Injektion von Furosemid wird eine endogene Renin-Ausschüttung angeregt. Anschließend werden die Testverbindungen durch kontinuierliche Infusion durch einmalige intravenöse Bolusgabe, durch intraduodenale oder durch perorale Gabe verabreicht und ihre Wirkung auf Blutdruck und Herzfrequenz wird gemessen. Die Verbindungen der vorliegenden Erfindung sind hierbei in einem Dosisbereich von etwa 0,1 - 5 mg/kg i.v. bzw. 1 - 50 mg/kg i.d. oder p.o. wirksam. Die in der vorliegenden Erfindung beschriebenen Verbindungen der allgemeinen Formel I können als Antihypertensiva sowie zur Behandlung der Herzinsuffizienz verwendet werden.

Die Erfindung betrifft daher auch die Verwendung von Verbindungen der Formel I als Heilmittel und pharmazeutische Zubereitungen, die diese Verbindung enthalten sowie ein Verfahren zu deren Herstellung. Bevorzugt ist die Anwendung bei Primaten, insbesondere beim Menschen.

Pharmazeutische Präparate enthalten eine wirksame Menge des Wirkstoffs der Formel I zusammen mit einem anorganischen oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose- oder Mannitlösungen oder auch eine Mischung aus den verschiedenen genannten Lösungsmitteln.

### Verzeichnis der verwendeten Abkürzungen:

- Boc: tert.-Butoxycarbonyl
- BuLi: n-Butyl-Lithium
- DC: Dünnschichtchromatographie
- DCC: Dicyclohexylcarbodiimid
- DCI: Desorption Chemical Ionisation
- DIP: Diisopropylether
- DNP: 2,4-Dinitrophenyl
- DME: Dimethoxyethan
- DMF: Dimethylformamid
- DOPA: 3,4-Dihydroxyphenylalanin
- EE: Essigsäureethylester
- EI: Electron Impact
- EtOC: Ethoxycarbonyl
- FAB: Fast atom bombardment
- H: n-Hexan
- HOBt: 1-Hydroxybenzotriazol
- Iva: Isovaleroyl
- M: Molekularpeak
- MeOH: Methanol
- MS: Massenspektrum
- MTB: Methyl-tert.-butylether
- NEM: N-Ethylmorpholin
- R.T.: Raumtemperatur
- THF: Tetrahydrofuran
- β-Val: 3-Amino-3-methyl-buttersäure

Die sonstigen für Aminosäuren verwendeten Abkürzungen entsprechen dem in der Peptidchemie üblichen drei Buchstaben-Code wie er z.B. in Europ. J. Biochem. 138, 9-37 (1984) beschrieben ist. Falls nicht ausdrücklich anders angegeben, handelt es sich immer um Aminosäuren der L-Konfiguration.

Die nachstehenden Beispiele dienen der Erläuterung der vorliegenden Erfindung.

### Beispiel 1

### a) 2-[(3S,4R,5S)-5-tert.-Butoxycarbonylamino-3,4-dihydroxy-6-cyclohexyl-hexyl]-pyridin

93 mg (1 mmol) 2-Picolin in 10 ml THF werden bei -78°C mit 1,4 ml (1 mmol) n-Buli versetzt. Nach Aufwärmen auf Raumtemperatur wird 30 min gerührt, dann auf -40°C abgekühlt. 1 mmol (2RS,3R,4S)-3-tert.-Butyldimethylsilyloxy-4-tert.-butoxycarbonylamino-5-cyclohexyl-1,2-oxopentan (bekannt aus EP-A 189 203, Beispiel 6) werden zugegeben (gelöst in 5 ml THF). Nach 10 Stunden bei Raumtemperatur wird mit Wasser verdünnt und mit MTB extrahiert. Das Rohprodukt (0,4 g) wird in THF gelöst und mit 5 ml einer 1 M Lösung von Tetrabutylammoniumfluorid in THF 1 h bei 0°C gerührt. Nach Verdünnen mit Wasser und Extrahieren mit Essigester erhält man 0,15 g des (3S,4R,5S)-Isomeren [MS (FAB): 391 (M + 1)] und 0,12 g des (3S,4S,5S)-Isomeren [MS (FAB): 391 (M + 1)].

### b) (3S,4R,5S)-2-[N-[Iva-Phe-His(DNP)]-5-amino-6-cyclohexyl-3,4-dihydroxy-hexyl]-pyridin

0,5 mmol des (3S,4R,5S)-Isomeren aus Beispiel 1a werden mit 5 ml HCl in DMF (gesättigt) 2 Stunden gerührt. Nach Einengen im Vakuum wird der Rückstand in 3 ml absolutem DMF gelöst. Je 0,5 mmol Iva-Phe-His(DNP)-OH, Dicyclohexylcarbodiimid und 1-Hydroxybenzotriazol werden zugegeben. Die Lösung wird mit N-Ethylmorpholin auf pH 9 gestellt und 24 Stunden gerührt. Nach Filtration wird mit EE verdünnt und je 1 Mal mit 3 %iger Natriumbicarbonatlösung, Wasser und gesättigter Kochsalzlösung gewaschen, mit Magnesiumsulfat getrocknet und eingeengt. Chromatographie an Kieselgel (MeOH/CH₂Cl₂ = 1 : 30) ergibt die Titelverbindung als gelbes Harz:
R_{f} (SiO₂; CH₂Cl₂/MeOH (10 : 1) = 0,6)
MS (FAB): 827 (M + 1)

### c) (3S,4R,5S)-2-[N-(Iva-Phe-His)-5-amino-6-cyclohexyl-3,4-dihydroxy-hexyl]-pyridin

0,1 g der Verbindung aus Beispiel 1b werden mit 30 mg Thiophenol in 2 ml Acetonitril 2 Stunden gerührt. Nach Einengen wird an Kieselgel mit CH₂Cl₂/MeOH/ges. NH₃ (10 : 1 : 0,1) chromatographiert. Man erhält 60 mg der Titelverbindung als Harz.
R_{f} (CH₂Cl : MeOH (10 : 1); SiO₂) : 0,05
MS (FAB): 661 (M + 1)
Unter Verwendung geeigneter Ausgangsmaterialien wurden analog der in vorstehenden Beispielen beschriebenen Verfahren hergestellt:

### Beispiel 2

### (3S,4R,5S)-2-[N-(Boc-Phe-His)-5-amino-6-cyclohexyl-3,4-dihydroxy-hexyl]-pyridin

MS (FAB): 677 (M + 1)

### Beispiel 3

### (3S,4R,5S)-2-[N-(EtOC-Phe-His)-5-amino-6-cyclohexyl-3,4-dihydro-hexyl]-pyridin

MS (FAB): 649 (M + 1)

### Beispiel 4

### (3S,4R,5S)-2-[N-(Iva-Phe-Nva)-5-amino-6-cyclohexyl-3,4-dihydroxy-hexyl]-pyridin

MS (FAB): 623 (M + 1)

### Beispiel 5

### (3S,4R,5S)-2-[N-(Iva-Phe-Nle)-5-amino-6-cyclohexyl-3,4-dihydroxy-hexyl]-pyridin

MS (FAB): 637 (M + 1)

### Beispiel 6

### (3S,4R,5S)-2-[N-(Iva-Phe-(NMe)-His)-5-amino-6-cyclohexyl-3,4-dihydroxy-hexyl]pyridin

MS (FAB): 675 (M + 1)

### Vergleichs-Beispiel 7

### a) 4(S)-Cyclohexylmethyl-5(R)-[3-(2-pyridyl)-1-(R,S)-hydroxy-propyl]-2-oxazolidinon

1 mmol 2-Picolin werden mit 1 mmol 4(S)-Cyclohexylmethyl-5(R)-[1(R,S)-2-oxoethyl]-2-oxazolidinon (bekannt aus EP-A 189 203, Beispiel 2) nach dem in Beispiel 1 angegebenen Verfahren umgesetzt. Chromatographie an SiO₂ mit EE/Cyclohexan (2 : 1) ergibt die beiden Diastereomeren (0,15 und 0,13 g).

### b) (3S,4R,5S)-2-[N-(Iva-Phe-His)-5-amino-6-cyclohexyl-3,4-dihydroxyl-hexyl]-pyridin

0,2 mmol 4(S)-Cyclohexylmethyl-5-(R)-[3-(2-pyridyl-1-(S)-hydroxy-propyl]-2-oxazolidinon (Beispiel 7a) werden mit 0,4 mmol Ba(OH)₂ · 8 H₂O in 4 ml Dioxan und 4 ml H₂O 9 Stunden am Rückfluß gekocht. Nach Verdünnen mit Dioxan wird abgesaugt und eingeengt. Das Rohprodukt wird, wie in Beispielen 1b und 1c beschrieben, weiter umgesetzt.

### Beispiel 8

### a) 1,1-Dibrom-3-cyclohexyl-1-propen

In einer Lösung von 0,2 Mol Cyclohexylacetaldehyd und 104,8 g Triphenylphosphin in 150 ml CH₂Cl₂ tropft man bei -10°C eine Lösung von 76,36 g Tetrabrommethan in 100 ml CH₂Cl₂. Nach 30 min Rühren bei Raumtemperatur wird abgesaugt, eingeengt und an Kieselgel mit Petrolether als Laufmittel gereinigt. Man erhält 48 g der Titelverbindung als Öl.
MS (EI): 292 (M⁺)

### b) 2-(6-Cyclohexyl-3-hydroxy-4-hexinyl)-pyridin

Eine Lösung von 0,1 Mol 1,1-Dibrom-3-cyclohexyl-1-propen in THF wird bei -78°C mit 2 Äquivalenten einer Lösung von n-Butyllithium in Hexan (1,4 M) versetzt. Nach Aufwärmen auf Raumtemperatur wird noch 1 Stunde gerührt, dann auf -78°C gekühlt und eine Lösung von 0,1 Mol 2-(2-Pyridyl)-propionaldehyd (hergestellt nach J. Pract. Chem. 19, 226 (1963)) zugegeben. Nach erneutem Aufwärmen auf Raumtemperatur wird auf Eis gegossen und mit MTB extrahiert. Nach Trocknen, Einengen und Chromatographie an Kieselgel (EE/Cyclohexan (1 : 1)) erhält man 20,4 g der Titelverbindung als Öl.
MS (EI): 257 (M⁺)

### c) E-2-(6-Cyclohexyl-3-hydroxy-4-hexenyl)-pyridin

136 ml (0,5 Mol) einer 70 %igen Lösung von Natrium-bis-methoxyethoxyaluminiumhydrid in Toluol werden mit 250 ml Ether verdünnt. Bei 0°C werden 0,312 Mol der Verbindung aus Beispiel 8b zugetropft. Nach 1 Stunde bei Raumtemperatur werden unter Kühlung 400 ml 2N H₂SO₄ zugetropft. Nach Aufarbeiten mit Ether wird mit MgSO₄ getrocknet und eingeengt.
MS (El): 259 (M⁺)

### d) 2-(6-Cyclohexyl-3-(S)-hydroxy-(4R,5S)-oxo-hexyl)-pyridin (A) und E-2-(6-Cyclohexyl-3(R)-hydroxy-4-hexenyl)-pyridin (B)

Zu einer Lösung von 0,1 Mol der Verbindung aus Beispiel 13 und 15 mmol L-(+)-Diisopropyltartrat in 480 ml abs. CH₂Cl₂ gibt man 10 g pulverisiertes Molekularsieb (3 Å). Bei -10°C werden 10 mmol Titan(IV)isopropylat zugetropft und 30 min gerührt. 23 ml (0,07 Mol) tert.-Butylhydroperoxid (3 M in Isooctan) werden zugetropft. Die Lösung wird in eine 0°C kalte Lösung von 73 g FeSO₄ · 7 H₂O und 11 g Citronensäure in 100 ml Wasser gegeben. Die wäßrige Phase wird mit Ether extrahiert. Die vereinigten organischen Phasen werden mit 10 ml einer Lösung von 5 g NaCl und 30 g NaOH in 90 ml Wasser 1 Stunde heftig gerührt. Nach Verdünnen mit H₂O wird mit MTB extrahiert, getrocknet und eingeengt. Das Rohprodukt wird an Kieselgel chromatographiert, wodurch A und B voneinander getrennt werden.

### e) 2-[(3S,4R,5R)-5-Amino-3,4-dihydroxy-6-cyclohexyl)-hexyl]-pyridin

1 mmol der Verbindung aus Beispiel 8d A werden in 5 ml Toluol zu einer Lösung von 1,2 mmol Diazidotitan(IV)diisopropylat in 10 ml Toluol bei 70°C gegeben. Nach 10 min wird abgekühlt und das Lösungsmittel entfernt. Der Rückstand wird in Et₂O aufgenommen und mit 8 ml 8 %iger H₂SO₄ 1 Stunde gerührt. Nach Extraktion mit CH₂Cl₂ wird mit MgSO₄ getrocknet und eingeengt. Das Rohprodukt wird in 20 ml MeOH mit Pd/C als Katalysator bei 1,1 bar und Raumtemperatur 2 Stunden hydriert. Man erhält die Titelverbindung als Öl.
MS (FAB): 299 (M + 1)

### f) (3S,4R,5R)-2-[N-(Iva-Phe-His)-5-amino-6-cyclohexyl-3,4-dihydroxy-hexyl]-pyridin

Diese Verbindung wird aus Beispiel 8e analog dem in Beispielen 1b und 1c angegebenen Verfahren durch Umsetzung mit Iva-Phe-His(DNP)OH und Thiophenol erhalten. Man erhält die Titelverbindung als blaßgelbes Harz.
MS (FAB): 661 (M + 1)

### Beispiel 9

### a) (3S,4R,5S)-2-[5-Amino-6-cyclohexyl-3,4-dihydroxy-hexyl]-pyridin · Hydrochlorid

12,9 mmol der Verbindung aus Beispiel 8d werden in 50 ml CH₂Cl₂ mit 4,6 ml Titan(IV)isopropylat bei Raumtemperatur 5 min gerührt. Eine Lösung von 3,79 g Lutidiniumtosylat in 50 ml CH₂Cl₂ wird zugegeben und 15 min gerührt. Nach Verdünnen mit Ether wird mit 80 ml 5 %iger Schwefelsäure 1 Stunde gerührt, mit CH₂Cl₂ extrahiert, mit MgSO₄ getrocknet und eingeengt. Das Rohprodukt wird mit 5 ml Dimethoxypropan und 40 mg p-Toluolsulfonsäure in 100 ml Toluol 3 Stunden am Wasserabscheider gekocht. Nach Abkühlen wird mit 1 N NaHCO₃-Lösung gewaschen und eingeengt. Der Rückstand wird in 30 ml DMF mit 5 Äquivalenten NaN₃ 3 Stunden bei 40°C gerührt. Nach Verdünnen mit Wasser wird mit MTB extrahiert. Der Extrakt wird 3 Mal mit Wasser gewaschen, mit MgSO₄ getrocknet und eingeengt. Das Rohprodukt wird in 30 ml Methanol mit Pd/C (10 %) als Katalysator bei Raumtemperatur und 1,1 bar H₂-Druck hydriert. Nach Filtration und Einengen wird der Rückstand in gesättigter HCl/DMF 30 min bei 20°C gerührt und die Lösung zur Trockne eingeengt.
MS (FAB): 292 (M + 1)

### b) BOC-His(DNP)-(3S,4R,5S)-2-[5-amino-6-cyclohexyl-3,4-dihydroxy-hexyl]-pyridin

Diese Verbindung wird hergestellt nach dem in Beispiel 1b angegebenen Verfahren durch Umsetzung von (3S,4R,5S)-2-[5-Amino-6-cyclohexyl-3,4-dihydroxy-hexyl]-pyridin (Beispiel 9a) mit BOC-His(DNP)OH.
MS (FAB): 696 (M + 1)

### c) H-His-(DNP)-(3S,4R,5S)-2-(5-Amino-6-cyclohexyl-3,4-dihydroxy-hexyl)-pyridinhydrochlorid

400 mg der Verbindung aus Beispiel 9b werden in 10 ml DME/HCl (gesättigt) 2 Stunden gerührt. Nach Einengen wird 2 Mal in Toluol aufgenommen und jeweils wieder eingeengt. Man erhält die Titelverbindung als gelben Schaum.
MS (FAB): 596 (M + 1)

### d) N-(2(S)-Benzyl-tert.-butylsulfonyl-propionyl)-His-(3S,4R,5S)-2-(5-amino-6-cyclohexyl-3,4-dihydroxy-hexyl)-pyridin

Je 0,8 mmol der Verbindung aus Beispiel 9c, 2(S)-Benzyl-3-tert.-butylsulfonyl-propionsäure (bekannt aus EP-A 236 734), 1 Hydroxybenztriazol und Dicyclohexylcarbodiimid werden in 3 ml DMF gelöst. Der pH-Wert wird mit N-Ethylmorpholin auf 9 eingestellt und 24 Stunden gerührt.

Der ausgefallene Dicyclohexylharnstoff wird abfiltriert, das Filtrat mit EE verdünnt und je 1 Mal mit 1 N NaHCO₃-Lösung, Wasser und gesättigter Kochsalzlösung gewaschen, mit MgSO₄ getrocknet und eingeengt. Das Rohprodukt wird in 3 ml Acetonitril gelöst und mit 45 mg Thiophenol 3 Stunden gerührt. Nach Einengen wird an Kieselgel mit CH₂Cl₂/MeOH/konz. NH₃ (10 : 1 : 0,1) chromatographiert. Man erhält die Titelverbindung als blaßgelbes Harz.
MS (FAB): 664 (M + 1)

### Beispiel 10

### a) 2-(6-Cyclohexyl-3-(R)-hydroxy-(4S,5R)-oxo-hexyl)-pyridin

Diese Verbindung erhält man aus E-2-(6-Cyclohexyl-3-(R)-hydroxy-4-hexenyl)-pyridin (Beispiel 8d B) nach dem in Beispiel 8d angegebenen Verfahren unter Verwendung von D-(-)-Weinsäurediisopropylester und 1,2 Äquivalenten tert.-Butylhydroperoxid.
MS (El): 275 (M⁺)

### b) 2-[(3R,4S,5S)-5-Amino-3,4-dihydroxy-6-cyclohexyl-hexyl]-pyridin

Diese Verbindung wird nach dem in Beispiel 8e angegebenen Verfahren aus der Verbindung aus Beispiel 10a hergestellt.

### c) (3R,4S,5S)-2-[N-(Iva-Phe-His)-5-amino-6-cyclohexyl-3,4-dihydroxy-hexyl]-pyridin

Diese Verbindung wird nach dem in Beispielen 1b und 1c angegebenen Verfahren aus der Verbindung aus Beispiel 10b hergestellt; farbloses Harz.
MS (FAB): 661 (M + 1)

### Beispiel 11

### a) (3R,4S,5R)-2-[5-Amino-6-cyclohexyl-3,4-dihydroxy-hexyl]-pyridin

Diese Verbindung wird nach dem in Beispiel 9a angegebenen Verfahren aus der Verbindung aus Beispiel 10a hergestellt.

### b) (3R,4S,5R)-2-[N-(Iva-Phe-His)-5-amino-6-cyclohexyl-3,4-dihydroxy-hexyl]-pyridin

Diese Verbindung wird nach dem in Beispielen 1a und 1b angegebenen Verfahren aus der Verbindung aus Beispiel 11a hergestellt.
MS (FAB): 661 (M + 1)
Analog der in Beispiel 9d angegebenen Vorschrift werden unter Verwendung geeigneter Ausgangsmaterialien hergestellt:

### Beispiel 12

### N-[2-(S)-(2-Thienyl-methyl)-3-tert.-butylsulfonyl-propionyl]-His-(3S,4R,5S)-2-(5-amino-6-cyclohexyl-3,4-dihydroxy-hexyl)-pyridin

MS (FAB): 670 (M + 1)

### Beispiel 13

### N-[2-(S)-(1-Naphthyl-methyl)-3-tert.-butylsulfonyl-propionyl]-His-(3S,4R,5S)-2-(5-amino-6-cyclohexyl-3,4-dihydroxy-hexyl)-pyridin

MS (FAB): 714 (M + 1)

### Beispiel 14

### N-[2-(S)-(2-Phenyl-methyl-3-isobutylsulfonyl-propionyl]-His-(3S,4R,5S)-2-(5-amino-6-cyclohexyl-3,4-dihydroxy-hexyl)-pyridin

MS (FAB): 664 (M + 1)

### Beispiel 15

### N-[(2-(S)-Phenyl-methyl-3-tert.-butylsulfonyl-propionyl]-Nva-(3S,4R,5S)-2-(5-amino-6-cyclohexyl-3,4-dihydroxy-hexyl)-pyridin

MS (FAB): 626 (M + 1)

### Beispiel 16

### N-[(2-(S)-Phenyl-methyl-3-tert.-butylsulfonyl-propionyl]-3-pyrazolyl-alanyl-(3S,4R,5S)-2-(5-amino-6-cyclohexyl-3,4-dihydroxy-hexyl)-pyridin

MS (FAB): 664 (M + 1)

### Beispiel 17

### N-[Bis-(1-naphthyl-methyl)-acetyl]-His-(3S,4R,5S)-2-(5-amino-6-cyclohexyl-3,4-dihydroxy-hexyl)-pyridin

Hergestellt analog Beispiel 9d aus der Verbindung aus Beispiel 9c und Bis-(1-naphthyl-methyl)-essigsäure (bekannt aus EP-A 228 182).
MS (FAB): 766 (M + 1)

### Beispiel 18

### N-[Bis-(2-Fluorbenzyl)-acetyl]-His-(3S,4R,5S)-2-(5-amino-6-cyclohexyl-3,4-dihydroxy-hexyl)-pyridin

Hergestellt analog Beispiel 9d aus der Verbindung aus Beispiel 9c und Bis-(2-Fluorbenzyl)essigsäure (bekannt aus EP-A 252 727).
MS (FAB): 702 (M + 1)

### Beispiel 19

### N-[3-Morpholinocarbonyl-2-(1-naphthyl)-propionyl]-His-(3S,4R,5S)-2-(5-amino-6-cyclohexyl-3,4-dihydroxy-hexyl)-pyridin

Hergestellt analog Beispiel 9d aus der Verbindung aus Beispiel 9c und 3-Morpholinocarbonyl-2-(1-naphthyl)-propionsäure (bekannt aus EP-A 200 406)
MS (FAB): 739 (M + 1)

### Beispiel 20

### N-(2-Benzyl-5,5-dimethyl-4-oxo-hexanoyl)-His-(3S,4R,5S)-2-(5-amino-6-cyclohexyl-3,4-dihydroxy-hexyl)-pyridin

Hergestellt analog Beispiel 9d aus der Verbindung aus Beispiel 9c und 2-Benzyl-5,5-dimethyl-4-oxo-hexansäure (bekannt aus EP-A 184 550).
MS (FAB): 660 (M + 1)

### Beispiel 21

### N-[3-(Piperazin-1-yl)-carbonyl-2-(1-naphthyl)methyl-propionyl]-His-(3S,4R,5S)-2-(5-amino-6-cyclohexyl-3,4-dihydroxy-hexyl)-pyridin

Hergestellt analog Beispiel 9d aus der Verbindung aus Beispiel 9c und 3-(4-Boc-piperazin-1-yl)-carbonyl-2-(1-naphthyl)methyl-propionsäure (bekannt aus EP-A 278 158) und nachfolgende Abspaltung der BOC-Gruppe mit Trifluoressigsäure.
MS (FAB): 715 (M + 1)

### Beispiel 22

### a) H-Phe-His(DNP)-(3S,4R,5S)-2-(5-amino-6-cyclohexyl-3,4-dihydroxy-hexyl)-pyridin-trifluoracetat

1 mmol der Verbindung aus Beispiel 9c wird zusammen mit je 1 mmol Boc-Phe-OH, 1-Hydroxybenzotriazol und Dicyclohexylcarbodiimid in 4 ml DMF gelöst; die Lösung wird mit N-Ethylmorpholin auf pH 9 gestellt und 24 Stunden gerührt. Nach Abfiltrieren des ausgefallenen Dicyclohexylharnstoffs wird mit Essigester verdünnt und je 1 Mal mit 1 N NaHCO₃-Lösung, Wasser und gesättigte Kochsalzlösung gewaschen, mit MgSO₄ getrocknet und eingeengt. Das Rohprodukt wird in 2 ml Trifluoressigsäure gelöst und 30 min gerührt. Nach Einengen erhält man die Titelverbindung als gelben Schaum.
MS (FAB): 577 (M + 1)

### b) N-[3-(3-Pyridyl)-propionyl]-Phe-His-(3S,4R,5S)-2-(5-amino-6-cyclohexyl-3,4-dihydroxy-hexyl)-pyridin

Hergestellt aus der Verbindung aus Beispiel 22a nach dem in Beispiel 9d angegebenen Verfahren durch Umsetung mit 3-(3-Pyridyl)-propionsäure.
MS (FAB): 720 (M + 1)

### Beispiel 23

### β-Val-Phe-His-(3S,4R,5S)-2-(5-amino-6-cyclohexyl-3,4-dihydroxy-hexyl)-pyridin

Hergestellt aus der Verbindung aus Beispiel 22a durch Umsetzung mit N-Benzyloxycarbonyl-3-amino-3-methylbuttersäure (bekannt aus EP-A 258 289) nach dem in Beispiel 9d angegebenen Verfahren und anschließender hydrogenolytischen Abspaltung der Benzyloxycarbonyl-Schutzgruppe mit H₂; Pd/C (10 %) in Essigsäure.
MS (FAB): 664 (M + 1)

### Beispiel 24

### N-Morpholinocarbonyl-Phe-His-(3S,4R,5S)-2-(5-amino-6-cyclohexyl-3,4-dihydroxy-hexyl)-pyridin

Hergestellt aus der Verbindung aus Beispiel 9c und N-Morpholinocarbonyl-Phe-OH (bekannt aus EP-A 258 289) nach dem in Beispiel 9d angegebenen Verfahren.
MS (FAB): 678 (M + 1)

### Beispiel 25

### a) 2-[(3S,4R,5S)-5-tert.-Butyloxycarbonylamino-3,4-dihydroxy-7-methyl-octyl]-pyridin

Hergestellt nach dem in Beispiel 1 angegebenen Verfahren ausgehend von (2RS,3R,4S)-3-tert.-Butyldimethylsilyl-4-tert.-butoxycarbonylamino-6-methyl-1,2-oxoheptan, das analog der in Beispiel 1 verwendeten Ausgangssubstanz aus Boc-leucinal zugänglich ist.
MS (FAB): 353 (M + 1)

### b) Iva-Phe-His-(3S,4R,5S)-2-(5-amino-3,4-dihydroxy-7-methyl-octyl)-pyridin

Hergestellt nach den in Beispielen 1b und 1c angegebenen Verfahren unter Verwendung der Verbindung aus Beispiel 25a als Ausgangsmaterial.
MS (FAB): 601 (M + 1)

### Beispiel 26

### N-(2-(S)-Benzyl-3-tert.-butylsulfonyl-propionyl)-His-(3S,4R,5S)-2-(5-amino-3,4-dihydroxy-7-methyl-octyl)-pyridin

Hergestellt nach den in Beispielen 9b - 9d angegebenen Verfahren aus der Verbindung aus Beispiel 25a.
MS (FAB): 576 (M + 1)

### Vergleichs Beispiel 27

### a) 2-[(3S,4R,5S)-5-tert.-Butoxycarbonylamino-6-cyclohexyl-3,4-dihydroxy-hexyl]-1-methyl-imidazol

Hergestellt analog der in Beispiel 1 angegebenen Vorschrift unter Verwendung von 1,2-Dimethylimidazol als Nucleophil.
MS (FAB): 382 (M + 1)

### b) Iva-Phe-His-(3S,4R,5S)-2-(5-Amino-6-cyclohexyl-3,4-dihydroxy-hexyl)-1-methyl-imidazol

Hergestellt aus der Verbindung aus Beispiel 27a analog den in Beispiel 1b und 1c angegebenen Verfahren.
MS (FAB): 664 (M + 1)

### Vergleichs-Beispiel 28

### a) 2-[(3S,4R,5S)-5-tert.-Butoxycarbonylamino-6-cyclohexyl-3,4-dihydroxy-hexyl]-4-(S)-1(S)-methyl-propyl-1,3-oxazolin

Hergestellt analog der in Beispiel 1 angegebenen Vorschrift unter Verwendung von 2-Lithio-methyl-4-(S)-1(S)-methyl-propyl-oxazolin als Nucleophil.
MS (FAB): 441 (M + 1)

### b) Iva-Phe-His-(3S,4R,5S)-2-(5-amino-6-cyclohexyl-3,4-dihydroxy-hexyl)-4-(S)-1(S)-methyl-propyl-1,3-oxazolin

Hergestellt analog den in Beispielen 1b und 1c angegebenen Vorschriften aus der Verbindung aus Beispiel 28.
MS (FAB): 708 (M + 1)

### Beispiel 29

### N-[3-Morpholinocarbonyl)2-phenyl-propionyl]-His-(3S,4R,5S)-(5-amino-6-cyclohexyl-3,4-dihydroxyhexyl)-pyridin

MS (FAB): 689 (M+1)

### Beispiel 30

### N-[3-Morpholinothiocarbonyl-2-phenyl-propionyl]-His-(3S,4R,5S)-2-(5-amino-6-cyclohexyl-3,4-dihydroxy-hexyl)-pyridin

MS (FAB): 705(M+1)

### Beispiel 31

### N-[3-Morpholinocarbonyl-2-(4-methoxy-phenyl)-propionyl]-His-(3S,4R,5S)-2-(5-amino-6-cyclohexyl-3-4-dihydroxy-hexyl)-pyridin

MS (FAB): 719 (M+1)

### Beispiel 32

### β-Val-O-methyl-tyrosyl-His-(3S,4R,5S)-2-(5-amino-6-cyclohexyl-3,4-dihydroxy-hexyl)-pyridin

MS (FAB): 694 (M+1)

### Beispiel 33

### N-Morpholinocarbonyl-O-methyl-tyrosyl-His-(3S,4R,5S)-2-(5-amino-6-cyclohexyl-3,4-dihydroxy-hexyl)-pyridin

MS (FAB): 708 (M+1)

### Beispiel 34

### N-Morpholinothiocarbonyl-Phe-His-(3S,4R,5S)-2-(5-amino-6-cyclohexyl-3,4-dihydroxy-hexyl)-pyridin

MS (FAB): 694 (M+1)

### Beispiel 35

### 4-Aminobutyryl-Phe-His-(3S,4R,5S)-2-(5-amino-6-cyclohexyl-3,4-dihydroxy-hexyl)-pyridin

MS (FAB): 650 (M+1)

### Beispiel 36

### 5-Aminovaleroyl-Phe-His-(3S,4R,5S)-2-(5-amino-6-cyclohexyl-3,4-dihydroxy-hexyl)-pyridin

MS (FAB): 664 (M+1)

### Beispiel 37

### 4-Aminobutyryl-O-methyl-tyrosyl-His-(3S,4R,5S)-2-(5-amino-6-cyclohexyl-3,4-dihydroxy-hexyl)-pyridin

MS (FAB): 680 (M+1)

### Beispiel 38

### 5-Aminovaleroyl-O-methyl-tyrosyl-His-(3S,4R,5S)-2-(5-amino-6-cyclohexyl-3,4-dihydroxy-hexyl)-pyridin

MS (FAB): 694 (M+1)

### Beispiel 39

### β-Val-Phe-(NMe)-His-(3S,4R,5S)-2-(5-amino-6-cyclohexyl-3,4-dihydroxy-hexyl)-pyridin

MS (FAB): 678 (M+1)

### Beispiel 40

### N-Morpholinocarbonyl-Phe-(NMe)-His-(3S,4R,5S)-2-(5-amino-6-cyclohexyl-3,4-dihydroxy-hexyl)-pyridin

MS (FAB): 692 (M+1)

### Beispiel 41

### 5-Aminovaleroyl-Phe-(NMe)-His-(3S,4R,5S)-2-(5-amino-6-cyclohexyl-3,4-dihydroxy-hexyl)pyridin

MS (FAB): 678 (M+1)

## Patentansprüche (Patentansprüche für folgende(n) Vertragsstaat(en): AT, BE, CH, DE, FR, GB, IT, LI, LU, NL, SE)

1. Verbindungen der Formel I 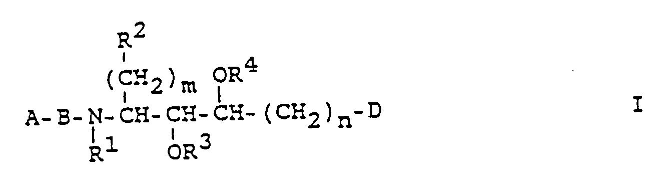 in welcher A einen Rest der Formel II 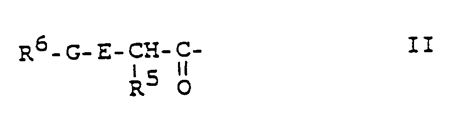 bedeutet, worin
R⁵ Phenyl, 2-Thienyl, 2-Pyridyl, 1-Naphthyl oder Benzyl, 2-Thienylmethyl, 2-Pyridylmethyl, 1-Naphthylmethyl, die jeweils gegebenenfalls durch Hydroxy, Dihydroxy, Methoxy, Dimethoxy, F oder Cl substituiert sind, bedeutet;
E eine CH₂-Gruppe, eine -NH-Gruppe oder eine N-(CH₃)-Gruppe bedeutet;
G einen Rest der Gruppe S, SO, SO₂, O, CO oder CS bedeutet;
R⁶ Methyl, Ethyl, Isopropyl, tert.-Butyl, Isobutyl, 2-Hydroxyethyl, 2-Methoxyethyl, Carboxymethyl, 2-Carboxyethyl, Methoxycarbonylmethyl, 2-Methoxycarbonylethyl, Ethoxycarbonylmethyl, 2-Ethoxycarbonylethyl, Carbamoylmethyl, 2-Carbamoylethyl, 2-Aminoethyl, 2-Dimethylaminoethyl, 2-Morpholinoethyl, Aminopropyl, Aminoisobutyl, Methylaminosobutyl, Dimethylaminoisobutyl, 2-Piperidinoethyl, Cyclopropyl, Cyclobutyl, Cyclopentyl oder Cyclohexyl, Phenyl, 1- oder 2-Naphthyl, o-, m- oder p-Methylphenyl, o-, m- oder p-Hydroxyphenyl oder o-, m- oder p-Aminophenyl, Benzyl, 2-Phenylethyl oder α- oder β-Naphthylmethyl, unsubstituiertes oder substituiertes Heteroaryl, z.B. 2- oder 3-Pyrrolyl, 2-Furyl, 2-Thienyl, 2- oder 4-Imidazolyl, 1-Methyl-2-, -4- oder -5-Imidazolyl, 1,3-Thiazol-2-yl, 2-, 3- oder 4-Pyridyl, 1-Oxido-2-, 3- oder 4-pyridinio, 2-Pyrazinyl, 2-, 4- oder 5-Pyrimidinyl, 2-, 3- oder 4-Chinolyl, 1-, 3- oder 4-Isochinolyl oder 2-Benzoxazolyl, Methoxy, Ethoxy oder n-Butoxy oder Amino als Teil eines fünf- oder sechsgliedrigen Ringes enthaltend ein Stickstoffatom und gewünschtenfalls ein Sauerstoffatom, z.B. 1-Pyrrolidinyl, 1-Piperidinyl oder 4-Morpholinyl, wobei der heterocyclische Ring auch durch ein oder zwei Reste der Gruppe (C₁-C₄)-Alkyl, (C₁-C₄)-Alkoxy, (C₁-C₄)-Alkoxycarbonyl, Hydroxy, Halogen, Amino, Mono- oder Di-(C₁-C₄)-alkylamino substituiert sein kann
B für einen bivalenten Rest aus der Gruppe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, 2,4-Diaminobuttersäure, Arginin, Norvalin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norleucin, Valin, Alanin, Cystein, S-Methylcystein, N-Methyl-Histidin, Benzodioxol-5-yl-alanin, 1,2,3,4-Tetrahydroisochinolin-3-carbonsäure, Homophenylalanin, 2-Amino-4-(2-thienyl)-buttersäure, (Z)-Dehydrophenylalanin, (E)-Dehydrophenylalanin, Dioxolan-1,3-yl-3-alanin steht;
R¹ Wasserstoff bedeutet;
R² Isopropyl oder Cyclohexyl bedeutet;
R³ und R⁴ gleich oder verschieden und voneinander unabhängig Wasserstoff, Acetoxymethyl, Acetoxyethyl, Pivaloyloxymethyl, Pivaloyloxyethyl, 2,2-Dimethylbutyryloxymethyl, Ethoxycarbonyloxymethyl, Ethoxycarbonyloxyethyl, tert.-Butoxycarbonyloxymethyl oder tert.-Butoxycarbonyloxyethyl bedeutet oder worin
R³ und R⁴ zusammen mit den sie tragenden Sauerstoffatomen einen Dioxolanon-, Dioxandion-, Dimethyldioxolan-, Phenyldioxolan- oder Cyclohexylidendioxolanring bilden;
D einen 2-, 3- oder 4-Pyridinrest,
m 1 bedeutet und
n 0, 1, 2 oder 3 bedeutet,
sowie deren physiologisch verträgliche Salze.

2. Verfahren zur Herstellung von Verbindungen der Formel I des Anspruchs 1 , dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

3. Verbindung gemäß Anspruch 1 zur Verwendung als Heilmittel.

4. Verbindung gemäß Anspruch 1 zur Verwendung als Heilmittel bei der Behandlung des Bluthochdrucks.

5. Pharmazeutisches Mittel enthaltend eine Verbindung gemäß Anspruch 1.

## Patentansprüche (Patentansprüche für folgende(n) Vertragsstaat(en): ES, GR)

1. Verfahren zur Herstellung von Verbindungen der Formel I 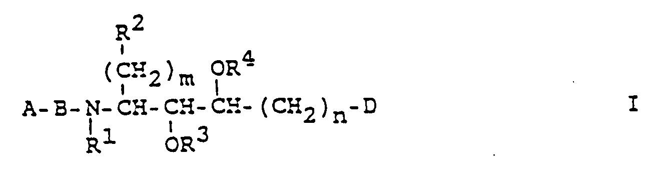 in welcher A einen Rest der Formel II 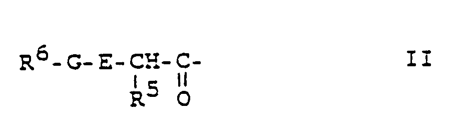 bedeutet, worin
R⁵ Phenyl, 2-Thienyl, 2-Pyridyl, 1-Naphthyl oder Benzyl, 2-Thienylmethyl, 2-Pyridylmethyl, 1-Naphthylmethyl, die jeweils gegebenenfalls durch Hydroxy, Dihydroxy, Methoxy, Dimethoxy, F oder Cl substituiert sind, bedeutet;
E eine CH₂-Gruppe, eine -NH-Gruppe oder eine N-(CH₃)-Gruppe bedeutet;
G einen Rest der Gruppe S, SO, SO₂, O, CO oder CS bedeutet;
R⁶ Methyl, Ethyl, Isopropyl, tert.-Butyl, Isobutyl, 2-Hydroxyethyl, 2-Methoxyethyl, Carboxymethyl, 2-Carboxyethyl, Methoxycarbonylmethyl, 2-Methoxycarbonylethyl, Ethoxycarbonylmethyl, 2-Ethoxycarbonylethyl, Carbamoylmethyl, 2-Carbamoylethyl, 2-Aminoethyl, 2-Dimethylaminoethyl, 2-Morpholinoethyl, Aminopropyl, Aminoisobutyl, Methylaminosooutyl, Dimethylaminoisobutyl, 2-Piperidinoethyl, Cyclopropyl, Cyclobutyl, Cyclopentyl oder Cyclohexyl, Phenyl, 1- oder 2-Naphthyl, o-, m- oder p-Methylphenyl, o-, m- oder p-Hydroxyphenyl oder o-, m- oder p-Aminophenyl, Benzyl, 2-Phenylethyl oder α- oder β-Naphthylmethyl, unsubstituiertes oder substituiertes Heteroaryl, z.B. 2- oder 3-Pyrrolyl, 2-Furyl, 2-Thienyl, 2- oder 4-Imidazolyl, 1-Methyl-2-, -4- oder -5-Imidazolyl, 1,3-Thiazol-2-yl, 2-, 3- oder 4-Pyridyl, 1-Oxido-2-, 3- oder 4-pyridinio, 2-Pyrazinyl, 2-, 4- oder 5-Pyrimidinyl, 2-, 3- oder 4-Chinolyl, 1-, 3- oder 4-Isochinolyl oder 2-Benzoxazolyl, Methoxy, Ethoxy oder n-Butoxy oder Amino als Teil eines fünf- oder sechsgliedrigen Ringes enthaltend ein Stickstoffatom und gewünschtenfalls ein Sauerstoffatom, z.B. 1-Pyrrolidinyl, 1-Piperidinyl oder 4-Morpholinyl, wobei der heterocyclische Ring auch durch ein oder zwei Reste der Gruppe (C₁-C₄)-Alkyl, (C₁-C₄)-Alkoxy, (C₁-C₄)-Alkoxycarbonyl, Hydroxy, Halogen, Amino, Mono- oder Di-(C₁-C₄)-alkylamino substituiert sein kann
B für einen bivalenten Rest aus der Gruppe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, 2,4-Diaminonuttersäure, Arginin, Norvalin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norleucin, Valin, Alanin, Cystein, S-Methylcystein, N-Methyl-Histidin, Benzodioxol-5-yl-alanin, 1,2,3,4-Tetahydroisocochinolin-3-carbonsäure, Homophenylalanin, 2-Amino-4-(2-thienyl)-buttersäure, (Z)-Dehydrophenylalanin, (E)-Dehydrophenylalanin, Dioxolan-1,3-yl-3-alanin steht;
R¹ Wasserstoff bedeutet;
R² Isopropyl oder Cyclohexyl bedeutet;
R³ und R⁴ gleich oder verschieden und voneinander unabhängig Wasserstoff, Acetoxymethyl, Acetoxyethyl, Pivaloyloxymethyl, Pivaloyloxyethyl, 2,2-Dimethylbutyryloxymethyl, Ethoxycarbonyloxymethyl, Ethoxycarbonyloxyethyl, tert.-Butoxycarbonyloxymethyl oder tert.-Butoxycarbonyloxyethyl bedeutet oder worin
R³ und R⁴ zusammen mit den sie tragenden Sauerstoffatomen einen Dioxolanon-, Dioxandion-, Dimethyldioxolan-, Phenyldioxolan- oder Cyclohexylidendioxolanring bilden;
D einen 2-, 3- oder 4-Pyridinrest,
m 1 bedeutet und
n 0, 1, 2 oder 3 bedeutet,
sowie deren physiologisch verträglichen Salze,
dadurch gekennzeichnet, daß man
ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

2. Verfahren zur Herstellung einer pharmazeutischen Zubereitung enthaltend eine nach einem der Verfahren des Anspruch 1 hergestellten Verbindung der Formel I, dadurch gekennzeichnet, daß man diese zusammen mit einem physiologisch unbedenklichen Träger und gegebenenfalls weiteren Hilfs- und Zusatzstoffen in eine geeignete Darreichungsform bringt.

## Claims (Claims for the following Contracting State(s): AT, BE, CH, DE, FR, GB, IT, LI, LU, NL, SE)

1. A compound of the formula I 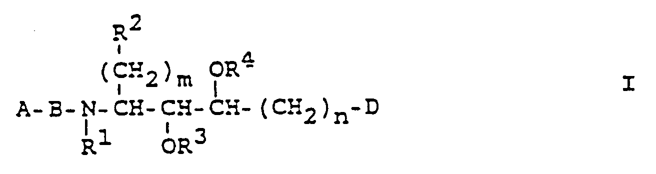 in which A denotes a radical of the formula II 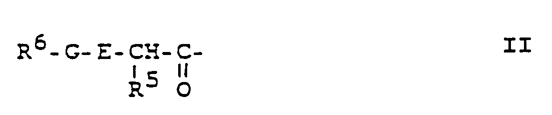 in which
R⁵ denotes phenyl, 2-thienyl, 2-pyridyl, 1-naphthyl or benzyl, 2-thienylmethyl, 2-pyridylmethyl or 1-naphthylmethyl which are in each case optionally substituted by hydroxyl, dihydroxy, methoxy, dimethoxy, F or Cl;
E denotes a CH₂ group, an -NH- group or an N-(CH₃)-group;
G denotes a radical from the group comprising S, SO, SO₂, O, CO or CS;
R⁶ denotes methyl, ethyl, isopropyl, tert-butyl, isobutyl, 2-hydroxyethyl, 2-methoxyethyl, carboxymethyl, 2-carboxyethyl, methoxycarbonylmethyl, 2-methoxycarbonylethyl, ethoxycarbonylmethyl, 2-ethoxycarbonylethyl, carbamoylmethyl, 2-carbamoylethyl, 2-aminoethyl, 2-dimethylaminoethyl, 2-morpholinoethyl, aminopropyl, aminoisobutyl, methylaminoisobutyl, dimethylaminoisobutyl, 2-piperidinoethyl, cyclopropyl, cyclobutyl, cyclopentyl or cyclohexyl, phenyl, 1- or 2-naphthyl, o-, m- or p-methylphenyl, o-, m- or p-hydroxyphenyl or o-, m- or p-aminophenyl, benzyl, 2-phenylethyl or α- or β-naphthylmethyl, unsubstituted or substituted heteroaryl, for example 2- or 3-pyrrolyl, 2-furyl, 2-thienyl, 2- or 4-imidazolyl, 1-methyl-2-, -4- or -5-imidazolyl, 1,3-thiazol-2-yl, 2-, 3- or 4-pyridyl, 1-oxido-2-, -3- or -4-pyridinio, 2-pyrazinyl, 2-, 4- or 5-pyrimidinyl, 2-, 3- or 4-quinolyl, 1-, 3- or 4-isoquinolyl or 2-benzoxazolyl, methoxy, ethoxy or n-butoxy or amino as part of a five- or six-membered ring containing a nitrogen atom and, if desired, an oxygen atom, for example 1-pyrrolidinyl, 1-piperidinyl or 4-morpholinyl, where the heterocyclic ring may also be substituted by one or two radicals from the group comprising (C₁-C₄)-alkyl, (C₁-C₄)-alkoxy, (C₁-C₄)-alkoxycarbonyl, hydroxyl, halogen, amino and mono- or di-(C₁-C₄)-alkylamino;
B stands for a bivalent radical from the group comprising phenylalanine, histidine, tyrosine, tryptophan, methionine, leucine, isoleucine, asparagine, aspartic acid, β-2-thienylalanine, β-3-thienylalanine, β-2-furylalanine, lysine, ornithine, 2,4-diaminobutyric acid, arginine, norvaline, 4-chlorophenylalanine, methionine sulfone, methionine sulfoxide, 2-pyridylalanine, 3-pyridylalanine, cyclohexylalanine, cyclohexylglycine, im-methylhistidine, O-methyltyrosine, O-benzyltyrosine, O-tert-butyltyrosine, phenylglycine, 1-naphthylalanine, 2-naphthylalanine, 4-nitrophenylalanine, norleucine, valine, alanine, cysteine, S-methylcysteine, N-methylhistidine, benzodioxol-5-yl-alanine, 1,2,3,4-tetrahydroisoquinoline-3-carboxylic acid, homophenylalanine, 2-amino-4-(2-thienyl)butyric acid, (Z)-dehydrophenylalanine, (E)-dehydrophenylalanine or 1,3-dioxolan-3-ylalanine;
R¹ denotes hydrogen;
R² denotes isopropyl or cyclohexyl;
R³ and R⁴ are identical or different and independently of one another denote hydrogen, acetoxymethyl, acetoxyethyl, pivaloyloxymethyl, pivaloyloxyethyl, 2,2-dimethylbutyryloxymethyl, ethoxycarbonyloxymethyl, ethoxycarbonyloxyethyl, tert-butoxycarbonyloxymethyl or tert-butoxycarbonyloxyethyl, or in which
R³ and R⁴ together with the oxygen atoms carrying them form a dioxolanone, dioxanedione, dimethyldioxolane, phenyldioxolane or cyclohexylidenedioxolane ring;
D denotes a 2-, 3- or 4-pyridine radical,
m denotes 1 and
n denotes 0, 1, 2 or 3,
and its physiologically tolerable salts.

2. A process for the preparation of a compound of the formula I of claim 1, which comprises coupling a fragment having a terminal carboxyl group or its reactive derivative with an appropriate fragment having a free amino group, optionally removing (a) protective group(s) temporarily introduced for the protection of other functional groups and optionally converting the compound thus obtained into its physiologically tolerable salt.

3. A compound as claimed in claim 1 for use as a pharmaceutical.

4. A compound as claimed in claim 1 for use as a pharmaceutical in the treatment of high blood pressure.

5. A pharmaceutical agent containing a compound as claimed in claim 1.

## Claims (Claims for the following Contracting State(s): ES, GR)

1. A process for the preparation of a compound of the formula I 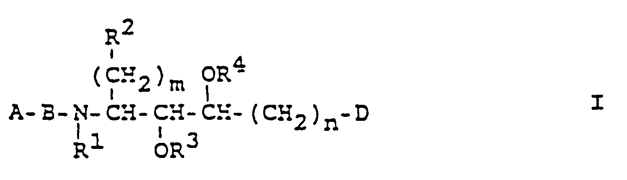 in which A denotes a radical of the formula II 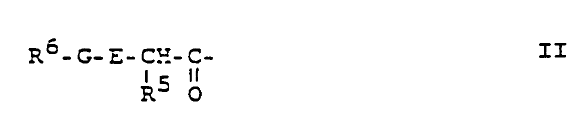 in which
R ⁵ denotes phenyl, 2-thienyl, 2-pyridyl, 1-naphthyl or benzyl, 2-thienylmethyl, 2-pyridylmethyl or 1-naphthylmethyl which are in each case optionally substituted by hydroxyl, dihydroxy, methoxy, dimethoxy, F or Cl;
E denotes a CH₂ group, an -NH- group or an N-(CH₃)-group;
G denotes a radical from the group comprising S, SO, SO₂, O, CO or CS;
R⁶ denotes methyl, ethyl, isopropyl, tert-butyl, isobutyl, 2-hydroxyethyl, 2-methoxyethyl, carboxymethyl, 2-carboxyethyl, methoxycarbonylmethyl, 2-methoxycarbonylethyl, ethoxycarbonylmethyl, 2-ethoxycarbonylethyl, carbamoylmethyl, 2-carbamoylethyl, 2-aminoethyl, 2-dimethylaminoethyl, 2-morpholinoethyl, aminopropyl, aminoisobutyl, methylaminoisobutyl, dimethylaminoisobutyl, 2-piperidinoethyl, cyclopropyl, cyclobutyl, cyclopentyl or cyclohexyl, phenyl, 1- or 2-naphthyl, o-, m- or p-methylphenyl, o-, m- or p-hydroxyphenyl or o-, m- or p-aminophenyl, benzyl, 2-phenylethyl or α- or β-naphthylmethyl, unsubstituted or substituted heteroaryl, for example 2- or 3-pyrrolyl, 2-furyl, 2-thienyl, 2- or 4-imidazolyl, 1-methyl-2-, -4- or -5-imidazolyl, 1,3-thiazol-2-yl, 2-, 3- or 4-pyridyl, 1-oxido-2-, -3- or -4-pyridinio, 2-pyrazinyl, 2-, 4- or 5-pyrimidinyl, 2-, 3- or 4-quinolyl, 1-, 3- or 4-isoquinolyl or 2-benzoxazolyl, methoxy, ethoxy or n-butoxy or amino as part of a five- or six-membered ring containing a nitrogen atom and, if desired, an oxygen atom, for example 1-pyrrolidinyl, 1-piperidinyl or 4-morpholinyl, where the heterocyclic ring may also be substituted by one or two radicals from the group comprising (C₁-C₄)-alkyl, (C₁-C₄)-alkoxy, (C₁-C₄)-alkoxycarbonyl, hydroxyl, halogen, amino and mono- or di-(C₁-C₄)-alkylamino;
B stands for a bivalent radical from the group comprising phenylalanine, histidine, tyrosine, tryptophan, methionine, leucine, isoleucine, asparagine, aspartic acid, β-2-thienylalanine, β-3-thienylalanine, β-2-furylalanine, lysine, ornithine, 2,4-diaminobutyric acid, arginine, norvaline, 4-chlorophenylalanine, methionine sulfone, methionine sulfoxide, 2-pyridylalanine, 3-pyridylalanine, cyclohexylalanine, cyclohexylglycine, im-methylhistidine, O-methyltyrosine, O-benzyltyrosine, O-tert-butyltyrosine, phenylglycine, 1-naphthylalanine, 2-naphthylalanine, 4-nitrophenylalanine, norleucine, valine, alanine, cysteine, S-methylcysteine, N-methylhistidine, benzodioxol-5-yl-alanine, 1,2,3,4-tetrahydroisoquinoline-3-carboxylic acid, homophenylalanine, 2-amino-4-(2-thienyl)butyric acid, (Z)-dehydrophenylalanine, (E)-dehydrophenylalanine or 1,3-dioxolan-3-ylalanine;
R¹ denotes hydrogen;
R² denotes isopropyl or cyclohexyl;
R³ and R⁴ are identical or different and independently of one another denote hydrogen, acetoxymethyl, acetoxyethyl, pivaloyloxymethyl, pivaloyloxyethyl, 2,2-dimethylbutyryloxymethyl, ethoxycarbonyloxymethyl, ethoxycarbonyloxyethyl, tert-butoxycarbonyloxymethyl or tert-butoxycarbonyloxyethyl, or in which
R³ and R⁴ together with the oxygen atoms carrying them form a dioxolanone, dioxanedione, dimethyldioxolane, phenyldioxolane or cyclohexylidenedioxolane ring;
D denotes a 2-, 3- or 4-pyridine radical,
m denotes 1 and
n denotes 0, 1, 2 or 3,
and of its physiologically tolerable salts, which comprises coupling a fragment having a terminal carboxyl group or its reactive derivative with an appropriate fragment having a free amino group, optionally removing (a) protective group(s) temporarily introduced for the protection of other functional groups and optionally converting the compound thus obtained into its physiologically tolerable salt.

2. A process for the production of a pharmaceutical preparation containing a compound of the formula I prepared by a process as claimed in claim 1, which comprises bringing said compound together with a physiologically acceptable excipient and, if desired, other suitable auxiliaries and additives into a suitable administration form.

## Revendications (Revendications pour l'(les) Etat(s) contractant(s) suivant(s): AT, BE, CH, DE, FR, GB, IT, LI, LU, NL, SE)

1. Composés de formule I 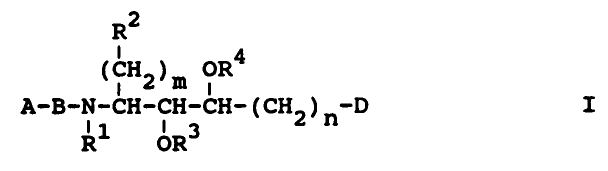 dans laquelle A représente un radical de formule II 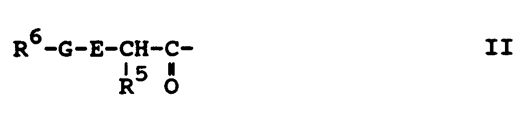 dans laquelle
R⁵ représente un radical phényle, 2-thiényle, 2-pyridyle, 1-naphtyle ou benzyle, 2-thiénylméthyle, 2-pyridylméthyle, 1-naphtylméthyle, qui sont chacun éventuellement substitués par un atome de F ou Cl ou par un groupe hydroxy, dihydroxy, méthoxy, diméthoxy;
E représente un groupe -CH₂-, un groupe -NH- ou un groupe -N-(CH₃)-;
G représente S, SO, SO₂, O, CO ou CS;
R⁶ représente un radical méthyle, éthyle, isopropyle, tert-butyle, isobutyle, 2-hydroxyéthyle, 2-méthoxyéthyle, carboxyméthyle, 2-carboxyéthyle, méthoxycarbonylméthyle, 2-méthoxycarbonyléthyle, éthoxycarbonylméthyle, 2-éthoxycarbonyléthyle, carbamoylméthyle, 2-carbamoyléthyle, 2-aminoéthyle, 2-diméthylaminoéthyle, 2-morpholinoéthyle, aminopropyle, aminoisobutyle, méthylaminoisobutyle, diméthylaminoisobutyle, 2-pipéridinoéthyle, cyclopropyle, cyclobutyle, cyclopentyle ou cyclohexyle, phényle, 1- ou 2-naphtyle, o-, m- ou p-méthylphényle, o-, m- ou p-hydroxyphényle ou o-, m- ou p-aminophényle, benzyle, 2-phényléthyle ou α- ou β-naphtylméthyle, un radical hétéroaryle substitué ou non substitué, par exemple 2- ou 3-pyrrolyle, 2-furyle, 2-thiényle, 2- ou 4-imidazolyle, 1-méthyl-2-, -4- ou -5-imidazolyle, 1,3-thiazol-2-yle, 2-, 3- ou 4-pyridyle, 1-oxydo-2-, -3- ou -4-pyridinio, 2-pyrazinyle, 2-, 4- ou 5-pyrimidinyle, 2-, 3- ou 4-quinolyle, 1-, 3- ou 4-isoquinolyle ou 2-benzoxazolyle, méthoxy, éthoxy ou n-butoxy, ou un groupe amino en tant que fragment d'un cycle à 5 ou 6 chaînons contenant un atome d'azote et, si on le désire, un atome d'oxygène, par exemple le groupe 1-pyrrolidinyle, 1-pipéridinyle ou 4-morpholinyle;
le cycle hétérocyclique pouvant également être substitué par un ou deux substituants choisis parmi des atomes d'halogène et des groupes alkyle en C₁-C₄, alcoxy en C₁-C₄, alcoxy(C₁-C₄)-carbonyle, hydroxy, amino, mono- ou dialkyl(C₁-C₄)-amino,
B représente un radical bivalent choisi parmi les restes phénylalanine, histidine, tyrosine, tryptophane, méthionine, leucine, isoleucine, asparagine, acide aspartique, β-2-thiénylalanine, β-3-thiénylalanine, β-2-furylalanine, lysine, ornithine, acide 2,4-diaminobutyrique, arginine, norvaline, 4-chlorophénylalanine, méthioninesulfone, méthioninesulfoxyde, 2-pyridylalanine, 3-pyridylalanine, cyclohexylalanine, cyclohexylglycine, im-méthylhistidine, O-méthyltyrosine, O-benzyltyrosine, O-tert-butyltyrosine, phénylglycine, 1-naphtylalanine, 2-naphtylalanine, 4-nitrophénylalanine, norleucine, valine, alanine, cystéine, S-méthylcystéine, N-méthylhistidine, benzodioxol-5-ylalanine, acide 1,2,3,4-tétrahydroisoquinoléine-3-carboxylique, homophénylalanine, acide 2-amino-4-(2-thiényl)butyrique, (Z)-déhydrophénylalanine, (E)-déhydrophénylalanine, dioxolanne-1,3-yl-3-alanine;
R¹ représente un atome d'hydrogène;
R² représente le groupe isopropyle ou cyclohexyle,;
R³ et R⁴ sont identiques ou différents et représentent, indépendamment l'un de l'autre, un atome d'hydrogène ou le groupe acétoxyméthyle, acétoxyéthyle, pivaloyloxyméthyle, pivaloyloxyéthyle, 2,2-diméthylbutyryloxyméthyle, éthoxycarbonyloxyméthyle, éthoxycarbonyloxyéthyle, tert-butoxycarbonyloxyméthyle ou tert-butoxycarbonyloxyéthyle, ou dans laquelle
R³ et R⁴ forment ensemble, avec les atomes d'oxygène qui les portent un cycle dioxolanone, dioxanedione, diméthyldioxolanne, phényldioxolanne ou cyclohexylidènedioxolanne;
D représente un reste 2-, 3- ou 4-pyridine;
m représente 1 et
n représente 0, 1, 2 ou 3,
et sels physiologiquement acceptables de ceux-ci.

2. Procédé pour la préparation des composés de formule I de la revendication 1, caractérisé en ce que l'on assemble un fragment à groupe carboxy terminal, ou un dérivé réactif de celui-ci, avec un fragment correspondant à groupe amino libre, on élimine un(des) groupe(s) protecteur(s) éventuellement introduit(s) temporairement pour la protection d'autres groupes fonctionnels, et éventuellement on convertit le composé obtenu en l'un de ses sels physiologiquement acceptable.

3. Composé selon la revendication 1, pour utilisation en tant que médicament.

4. Composé selon la revendication 1, pour utilisation en tant que médicament dans le traitement de l'hypertension artérielle.

5. Produit pharmaceutique contenant un composé selon la revendication 1.

## Revendications (Revendications pour l'(les) Etat(s) contractant(s) suivant(s): ES, GR)

1. Procédé pour la préparation de composés de formule I 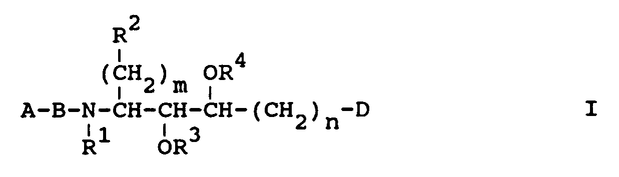 dans laquelle A représente un radical de formule II 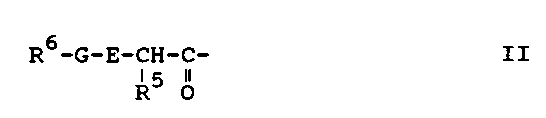 dans laquelle
R⁵ représente un radical phényle, 2-thiényle, 2-pyridyle, 1-naphtyle ou benzyle, 2-thiénylméthyle, 2-pyridylméthyle, 1-naphtylméthyle, qui sont chacun éventuellement substitués par un atome de F ou Cl ou par un groupe hydroxy, dihydroxy, méthoxy, diméthoxy;
E représente un groupe -CH₂-, un groupe -NH- ou un groupe -N-(CH₃)-;
G représente S, SO, SO₂, O, CO ou CS;
R⁶ représente un radical méthyle, éthyle, isopropyle, tert-butyle, isobutyle, 2-hydroxyéthyle, 2-méthoxyéthyle, carboxyméthyle, 2-carboxyéthyle, méthoxycarbonylméthyle, 2-méthoxycarbonyléthyle, éthoxycarbonylméthyle, 2-éthoxycarbonyléthyle, carbamoylméthyle, 2-carbamoyléthyle, 2-aminoéthyle, 2-diméthylaminoéthyle, 2-morpholinoéthyle, aminopropyle, aminoisobutyle, méthylaminoisobutyle, diméthylaminoisobutyle, 2-pipéridinoéthyle, cyclopropyle, cyclobutyle, cyclopentyle ou cyclohexyle phényle, 1- ou 2-naphtyle, o-, m- ou p-méthylphényle, o-, m- ou p-hydroxyphényle ou o-, m- ou p-aminophényle, benzyle, 2-phényléthyle ou α- ou β-naphtylméthyle, un radical hétéroaryle substitué ou non substitué, par exemple 2- ou 3-pyrrolyle, 2-furyle, 2-thiényle, 2- ou 4-imidazolyle, 1-méthyl-2-, -4- ou -5-imidazolyle, 1,3-thiazol-2-yle, 2-, 3- ou 4-pyridyle, 1-oxydo-2-, -3- ou -4-pyridinio, 2-pyrazinyle, 2-, 4- ou 5-pyrimidinyle, 2-, 3- ou 4-quinolyle, 1-, 3- ou 4-isoquinolyle ou 2-benzoxazolyle, méthoxy, éthoxy ou n-butoxy, ou un groupe amino en tant que fragment d'un cycle à 5 ou 6 chaînons contenant un atome d'azote et, si on le désire, un atome d'oxygène, par exemple le groupe 1-pyrrolidinyle, 1-pipéridinyle ou 4-morpholinyle;
le cycle hétérocyclique pouvant également être substitué par un ou deux substituants choisis parmi des atomes d'halogène et des groupes alkyle en C₁-C₄, alcoxy en C₁-C₄, alcoxy(C₁-C₄)-carbonyle, hydroxy, amino, mono- ou dialkyl(C₁-C₄)-amino,
B représente un radical bivalent choisi parmi les restes phénylalanine, histidine, tyrosine, tryptophane, méthionine, leucine, isoleucine, asparagine, acide aspartique, β-2-thiénylalanine, β-3-thiénylalanine, β-2-furylalanine, lysine, ornithine, acide 2,4-diaminobutyrique, arginine, norvaline, 4-chlorophénylalanine, méthioninesulfone, méthioninesulfoxyde, 2-pyridylalanine, 3-pyridylalanine, cyclohexylalanine, cyclohexylglycine, im-méthylhistidine, O-méthyltyrosine, O-benzyltyrosine, O-tert-butyltyrosine, phénylglycine, 1-naphtylalanine, 2-naphtylalanine, 4-nitrophénylalanine, norleucine, valine, alanine, cystéine, S-méthylcystéine, N-méthylhistidine, benzodioxol-5-ylalanine, acide 1,2,3,4-tétrahydroisoquinoléine-3-carboxylique, homophénylalanine, acide 2-amino-4-(2-thiényl)butyrique, (Z)-déhydrophénylalanine, (E)-déhydrophénylalanine, dioxolanne-1,3-yl-3-alanine;
R¹ représente un atome d'hydrogène;
R² représente le groupe isopropyle ou cyclohexyle,;
R³ et R⁴ sont identiques ou différents et représentent, indépendamment l'un de l'autre, un atome d'hydrogène ou le groupe acétoxyméthyle, acétoxyéthyle, pivaloyloxyméthyle, pivaloyloxyéthyle, 2,2-diméthylbutyryloxyméthyle, éthoxycarbonyloxyméthyle, éthoxycarbonyloxyéthyle, tert-butoxycarbonyloxyméthyle ou tert-butoxycarbonyloxyéthyle, ou dans laquelle
R³ et R⁴ forment ensemble, avec l'atome d'oxygène qui les porter, un cycle dioxolanone, dioxanedione, diméthyldioxolanne, phényldioxolanne ou cyclohexylidènedioxolanne;
D représente un reste 2-, 3- ou 4-pyridine;
m représente 1 et
n représente 0, 1, 2 ou 3,
ainsi que de leurs sels physiologiquement acceptables, caractérisé en ce que l'on assemble un fragment à groupe carboxy terminal, ou un dérivé réactif de celui-ci, avec un fragment correspondant à groupe amino libre, on élimine un(des) groupe(s) protecteur(s) éventuellement introduit(s) temporairement pour la protection d'autres groupes fonctionnels, et éventuellement on convertit le composé obtenu en l'un de ses sels physiologiquement acceptable.

2. Procédé pour la préparation d'une composition pharmaceutique contenant un composé préparé selon l'un des procédés de la revendication 1, caractérisé en ce qu'on le met sous une forme d'administration appropriée, conjointement avec un véhicule physiologiquement acceptable et éventuellement d'autres additifs et adjuvants.
